# EUROPEAN PATENT APPLICATION

(11) **EP 4 535 364 A1**
(43) Date of publication of application: **09.04.2025**
(21) Application number: 24203147.4
(22) Date of filing: 27.09.2024
(51) Int. Cl.: G16C 20/20, G16C 20/70

(54) **SCIENTIFIC EXPERIMENT METHOD CREATION**

(30) Priority: 05.10.2023 US 202363588067 P
(71) Applicant: Thermo Electron Manufacturing Limited, Hemel Hempstead Hertfordshire HP2 7GE (GB)
(72) Inventor: HADFIELD, Maximillian Spencer, Hemel Hempstead HP2 7GE (GB)
(74) Representative: Boult Wade Tennant LLP

(57) **Abstract**

Methods and systems for scientific method creation. One method includes building a data store including a plurality of linked data sets linking a predetermined experiment method defining a plurality of experiment parameters, a predetermined sample type, and a predetermined analysis; training a model with the plurality of linked data set stored in the data store; inputting to the model, as trained, at least one selected from a group consisting of a sample type and a desired analysis; and outputting, from the model, a list of one or more recommended methods based on the at least one of the sample and the desired analysis. The method may also include receiving actual experiment results collected via one or more scientific instructions, the actual experiment results associated with performance of a method from the list; determining expected results for the performed methods; and verifying the actual experiment results based on expected results.

## Description

### Related Applications

This application claims the benefit of U.S. Provisional Patent Application No. 63/588,067, filed October 5, 2023, the entire content of which is hereby incorporated by reference.

### Background

Scientific instruments may include a complex arrangement of movable components, sensors, input and output ports, energy sources, and consumable components. Lab systems may include many instruments and many individuals interacting with different instruments. Lab systems may support one or more scientific experiments based on a selection of one or more experiment parameters.

### Brief Description of the Drawings

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 is a block diagram of an example scientific instrument support system, in accordance with various embodiments.
FIG. 2 is a block diagram of an example scientific instrument support module for performing lab management operations, in accordance with various embodiments.
FIG. 3 is a block diagram showing functionality of method creator logic included in the support module of FIG. 2, in accordance with various embodiments.
FIG. 4 is an example graphical user interface ("GUI") provided as part of the lab management methods disclosed herein, in accordance with various embodiments.
FIG. 5 is a block diagram of an example computing device that may perform some or all of the scientific instrument support methods disclosed herein, in accordance with various embodiments.
FIG. 6 is a block diagram of an example scientific instrument support system in which some or all of the scientific instrument support methods disclosed herein may be performed, in accordance with various embodiments.
FIGS. 7 and 8 are flow diagrams of example methods of performing support operations, in accordance with various embodiments.
FIG. 9 is a block diagram schematically representing the methods of FIGS. 7 and 8 in accordance with various embodiments.

### Detailed Description

Disclosed herein are scientific instrument support systems, as well as related methods, computing devices, and computer-readable media. For example, in some embodiments, a support system is provided for establishing a cloud-based platform for one or more scientific instruments. The platform can identify what scientific instruments are connected, determine an availability of such instruments, manage a schedule of such instructions (through a reservation system), track projects used with the instruments, including, for example, methods applied by each instrument for a particular type of sample and associated results, and the like. For example, the platform can track and receive project information associated with the scientific instrument groups (including one or more instruments) connected to the platform, including sample information, the method (experiment parameters) applied by the scientific instrument groups, and the results of such methods (e.g., the project result, timing information, etc.), including data collected via the scientific instrument groups in raw form, processed form, or a combination thereof. For example, a networked instrument platform may include or interact with one or more data stores that stores methods associated with the networked instruments and associated information.

Given such a connected network and data tracking and collection, the data can be used, as described herein, to create a method performed by one or more analytical instruments to analyze a sample. For example, using machine learning techniques, data collected within the network platform can be used to train a model to (1) recommend a method (e.g., one or more experiment parameters defining an experiment performed via one or more analytical instruments) based on, for example, a sample type, a needed analysis (e.g., one or more compounds to be identified and/or quantified in the sample, a characteristic of the sample, or the like), or a combination thereof, and, in some embodiments, (2) provide an assessment of the resulting data against predicted data to efficiently assess whether the resulting data is correct and also to further train the model and improve subsequent method recommendations. Embodiments described herein may provide one or more user interfaces as part of the method creation. The user interfaces may provide a chat functionality where a user enters a sample type, a desired analysis (e.g., one or more compounds of interest), or a combination thereof and responses (e.g., a list of one or more methods) are provided to the user with prompts for further selections or input to narrow down or filter options from initial recommendations provided via the chat interaction. In some embodiments, the model can be trained using one sets of linked data, wherein each data set may associate a particular method (e.g., a set of experiment parameters) with a particular sample, and analysis (e.g., one or more compounds of interest), wherein the model learns to recommend a method for a particular sample and/or desired analysis and also optionally predict results that can be used to assess the quality of the data generated via the method. Feedback from the user regarding the recommendations and feedback from the results can also be provided to the model as further training data.

The connected network can also be used to filter methods recommended to a user to limit the recommendations to those methods used with instruments that are available (e.g., present in a laboratory, available for current use (e.g., based on reservation data, consumable inventories, etc.), or the like). Similarly, in some embodiments, the network may be configured to provide one or more recommended methods using available instruments, one or more recommended methods using one or more recommended instruments (which may not be available in the relevant laboratory), or a combination thereof and a user may be able to specify (through the setting one or more optimization settings or parameters configured through, for example, one or more user interfaces). A laboratory manager may use recommended methods that are not optimized for the available laboratory resources to make purchasing decisions for a laboratory (e.g., purchase instruments commonly used in recommended methods).

Similarly, the methods and systems described herein may act as a method converter that may use methods created for one instrument vendor to efficiently establish a method for a different vendor. For example, if an existing method for one vendor is associated with particular predicted results, these results may be used to build a similar method for a different vendor, such as, for example, by building an initial method from the existing method and interactively running the initial method on the vendor instruments (with modification between interactions) until the results from the method generally match the expected results.

Accordingly, the methods and systems described herein simplify and reduce the time and resources needed to configure a project (which can manually take days or months) that may otherwise limit the types of samples and analysis a laboratory can perform. The connected (e.g., cloud-based) platform enables data collection and linking to create appropriate training data for such methods and systems. Accordingly, methods and systems described herein improve productivity, reduce learning time, and reduce resource usage (human resources, laboratory resources, and computing resources).

The method created via the support systems and associated functionality described herein may define "how" an analysis will be performed on a sample and may include one or more experiment parameters, wherein the experiment parameters define, for example, an identification of one or more instruments, sample preparatory requirements, instrument and environment settings, regulatory factors, or a combination thereof, or a combination thereof. The experiment parameters may also define how the results of the method can be analyzed and checked, such as, for example, by specifying calibrations to use in the data processing, what expected results should be (within specified tolerances), what erroneous results look like, or a combination thereof. In some embodiments, the experiment parameters may include predicted results (which may include historical results or synthetic results) that can be compared to actual results from the method to determine whether the results are correct (and, optionally, whether the created method was correct). As noted above, comparisons between predicted results and actual results may also be used as feedback to the model for further training purposes.

As noted above, embodiments described herein achieve improved lab management and operation relative to conventional approaches. In particular, embodiments described herein reduce time and resources needed for the development of methods for scientific experiments and data review relative to conventional approaches, which improves overall operation of scientific laboratories. For example, some embodiments disclosed herein provide various user interfaces and associated computational processes that are unconventional in the field of lab management and development of scientific experiments and data review. The computational processes and user interface features disclosed herein do not only involve the collection and comparison of information but apply new analytical and technical techniques to create methods more efficiently and accurately than conventional approaches. The present disclosure thus introduces functionality that neither a conventional computing, nor a human, could perform.

Accordingly, the embodiments of the present disclosure may serve any of a number of technical purposes, such as developing scientific experiments faster and more efficiently in terms of resource usage, providing data review of scientific experimental results to verify data correctness, which may limit or avoid re-runs or other service issues, managing a specific technical system or process; determining machine or instrument availability or status; optimizing load distribution in a computer network (e.g., by distributing projects and associated data processing across instruments included in one or more labs); providing a faster processing of sensor data (e.g., by identifying optimized methods for various experiment parameters processing); or a combination thereof.
The embodiments disclosed herein thus provide improvements to development of scientific experiments, and lab and instrument management and usage and associated technology (e.g., improvements in the computer technology supporting scientific instruments, among other improvements).

In the following detailed description, reference is made to the accompanying drawings that form a part hereof wherein like numerals designate like parts throughout, and in which is shown, by way of illustration, embodiments that may be practiced. It is to be understood that other embodiments may be utilized, and structural or logical changes may be made, without departing from the scope of the present disclosure. Therefore, the following detailed description is not to be taken in a limiting sense.

Various operations may be described as multiple discrete actions or operations in turn, in a manner that is most helpful in understanding the subject matter disclosed herein. However, the order of description should not be construed as to imply that these operations are necessarily order dependent. In particular, these operations may not be performed in the order of presentation. Operations described may be performed in a different order from the described embodiment. Various additional operations may be performed, and/or described operations may be omitted in additional embodiments.

For the purposes of the present disclosure, the phrases "A and/or B" and "A or B" mean (A), (B), or (A and B). For the purposes of the present disclosure, the phrases "A, B, and/or C" and "A, B, or C" mean (A), (B), (C), (A and B), (A and C), (B and C), or (A, B, and C). Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices.

The description uses the phrases "an embodiment," "various embodiments," and "some embodiments," each of which may refer to one or more of the same or different embodiments. Furthermore, the terms "comprising," "including," "having," and the like, as used with respect to embodiments of the present disclosure, are synonymous. When used to describe a range of dimensions, the phrase "between X and Y" represents a range that includes X and Y. As used herein, an "apparatus" may refer to any individual device, collection of devices, part of a device, or collections of parts of devices. The drawings are not necessarily to scale.

Unless the context of their usage unambiguously indicates otherwise, the articles "a," "an," and "the" should not be interpreted as meaning "one" or "only one." Rather these articles should be interpreted as meaning "at least one" or "one or more." Likewise, when the terms "the" or "said" are used to refer to a noun previously introduced by the indefinite article "a" or "an," "the" and "said" mean "at least one" or "one or more" unless the usage unambiguously indicates otherwise.

It should also be understood that although certain drawings illustrate hardware and software located within particular devices, these depictions are for illustrative purposes only. In some embodiments, the illustrated components may be combined or divided into separate software, firmware, and/or hardware. For example, instead of being located within and performed by a single electronic processor, logic and processing may be distributed among multiple electronic processors. Regardless of how they are combined or divided, hardware and software components may be located on the same computing device or may be distributed among different computing devices connected by one or more networks or other suitable communication links.

Thus, in the claims, if an apparatus or system is claimed, for example, as including an electronic processor or other element configured in a certain manner, for example, to make multiple determinations, the claim or claim element should be interpreted as meaning one or more electronic processors (or other element) where any one of the one or more electronic processors (or other element) is configured as claimed, for example, to make some or all of the multiple determinations. To reiterate, those electronic processors and processing may be distributed.

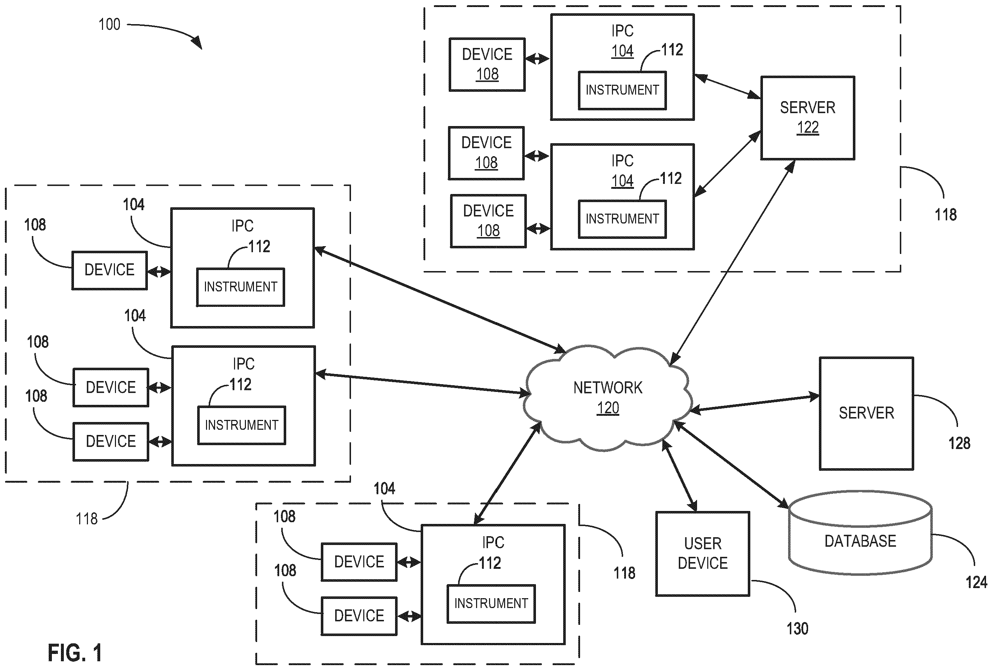

FIG. 1 is a block diagram of a scientific instrument support system 100 (henceforth referred to simply as support system 100) for performing scientific instrument support operations, in accordance with various embodiments. As illustrated in FIG. 1, the support system 100 includes a plurality of instrument personal computer (IPC)s 104 connected over a communication connection or network 120. One or more devices 108 are connected to each IPC 104. Devices 108 are physical entities that perform some function of sample preparation and/or sample analysis. For example, devices 108 may be physical devices having a serial number, a means of communicating with other external entities, and may include processors, memory, and firmware. Devices 108 may include, for example, sensors, detectors, actuators, spectrometers, spectrograms, oscilloscopes, electrometers, interferometers, and the like. The IPCs 104 may also include one or more instruments 112. Instruments 112 are logical containers that include a collection of devices 108. For example, devices 108 work together to perform operations and produce results. The logical collection of devices 108 (e.g., the instrument 112) prepares or analyzes inputs, such as blood samples or other biological samples, semiconductors, chemical compounds, solutions, food and drug samples, and the like. Each IPC 104 is connected to its respective device(s) 108 and may be configured to communicate unidirectionally or bi-directionally with the connected device(s) 108. Each IPC 104 may be connected to the device(s) 108 via wired or wireless communication mediums and/or protocols. Each IPC 104 may transmit commands to the connected device(s) 108, receive status signals from the device(s) 108, receive measurements from the device(s) 108, and the like, as described below in more detail. The IPCs 104, and their corresponding connected device(s) 108, may be organized into a plurality of scientific instrument groups 118. For example, a scientific instrument group 118 may include a liquid chromatography ("LC") instrument IPC 104 and a mass spectrometry ("MS") IPC 104. Each scientific instrument group 118 includes at least one IPC 104. The IPCs 104, the devices 108, and the instruments 112 may be more generally referred to as service components. The instruments 112 may include, for example, liquid chromatography instruments, gas chromatography instruments, ion chromatography instruments, mass spectrometry instruments, trace elemental instruments (e.g., inductively coupled plasma mass spectrometry, inductively coupled plasma optical emission spectroscopy, atomic absorption, etc.), capillary electrophoresis instruments, spectroscopy instruments, absorptiometry instruments, scattered radiation instruments, emitted radiation instruments, conductometry instruments, amperometry instruments, voltammetry instruments, electrogravimetry instruments, coulometry instruments, potentiometry instruments, and the like. However, it should be understood that instruments 112 are not limited to those described herein, and other types of instruments are contemplated. For example, any type of instrument configured to follow or interact with the defined user interfaces may be supported and may be automatically detected (as described below) and managed within the system 100. Furthermore, as also described in more detail below, in some embodiments, the system 100 is configured to enable a user to manually add an instrument for management within the system 100.

The support system 100 includes a database 124, a server 128, and a user device 130 communicatively coupled with the plurality of IPCs 104 (and with each other) through the communication network 120. In other embodiments, the plurality of IPCs 104, the server 128, and the database 124 communicate via one or more dedicated wire connections or other forms of wired or wireless electronic communication. It should be understood that the support system 100 may include fewer or additional components than those illustrated in FIG. 1. For example, the support system 100 may include fewer or more IPCs 104, multiple servers 128, multiple databases 124, or a combination thereof. In some instances, rather than including an independent database, the storage functionality of the database 124 is provided by the server 128. The components of the support system 100 may also communicate through one or more intermediary devices not illustrated in FIG. 1.

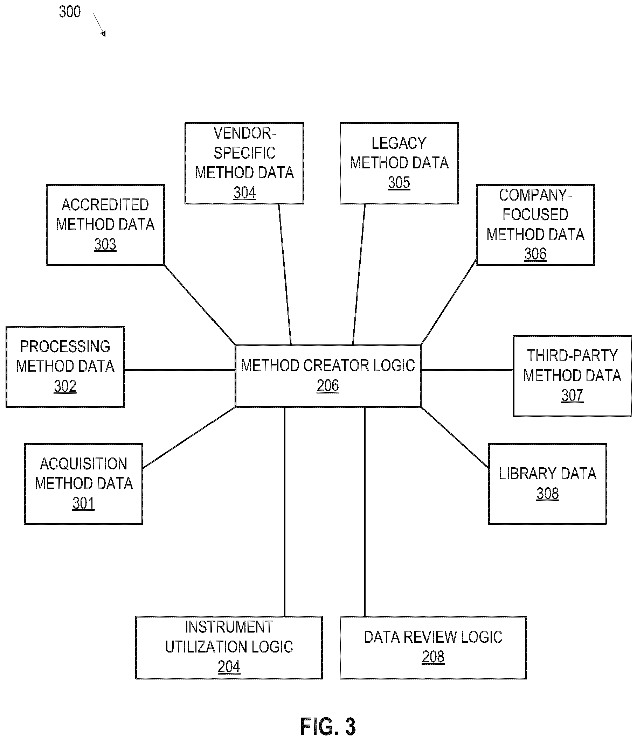

The server 128 serves as a "control hub" for the plurality of IPCs 104 and may establish a connected platform of the IPCs 104 and associated instruments 112 and devices 108. For example, the server 128 communicates with each of the IPCs 104 by sending commands to the IPCs 104 to perform methods described herein over a wired connection, a wireless connection, or a combination thereof. Additionally, the server 128 receives and/or manages measurements and statuses of device(s) 108 and instrument(s) 112 from their respective IPCs 104 over a wired connection, wireless connection, or a combination thereof. The database 124 stores data indicating the status of components within the support system 100. For example, the database 124 may store a current status of the device(s) 108 and instrument(s) 112, historical statuses of the device(s) 108 and instrument(s) 112, measurements recorded by the device(s) 108 and instrument(s) 112, and the like. The database 124 may also store data defining methods, samples, and analysis associated with the support system 100, such as, for example, acquisition method data 301, processing method data 302, accredited method data 303, vendor-specific method data 304, legacy method data 305, methods associated with a particular user or group of users (e.g., company-focused method data 306), methods pulled or scrapped from various third-party systems (e.g., journals, research papers, etc.), i.e., third-party data 307, or a combination thereof (see, e.g., FIG. 3). The database 124 may also store one or more libraries (i.e., library data 308), such as one or more spectral libraries, instrument geometries (e.g., column geometries), chromatography databases, etc.) (see, e.g., FIG. 3). Accordingly, in some embodiments, the database 124 may be referred to as an analytical data store and, as noted above, may include one data storage device or multiple data storage devices.

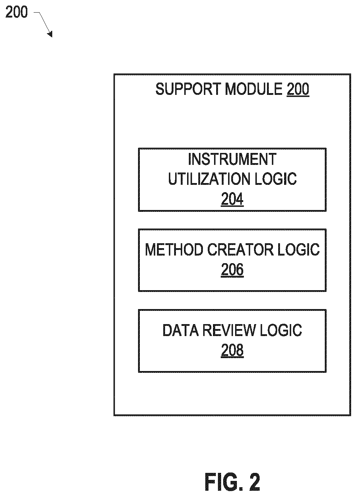

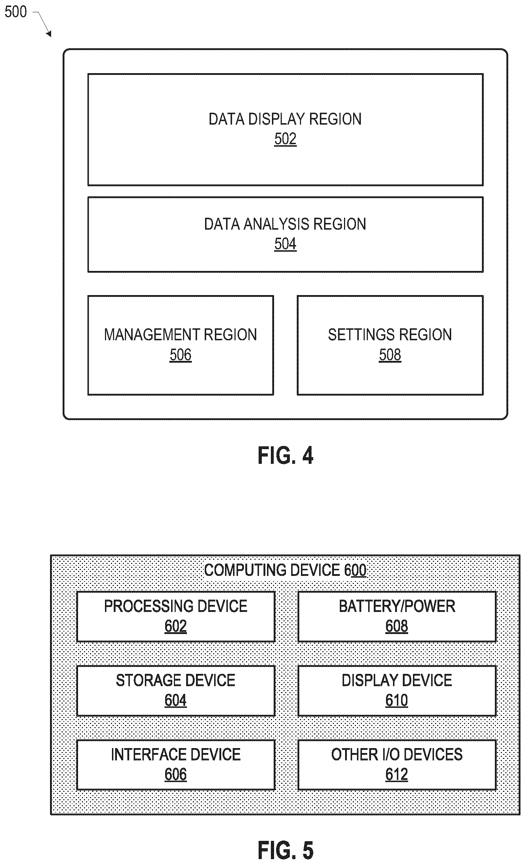

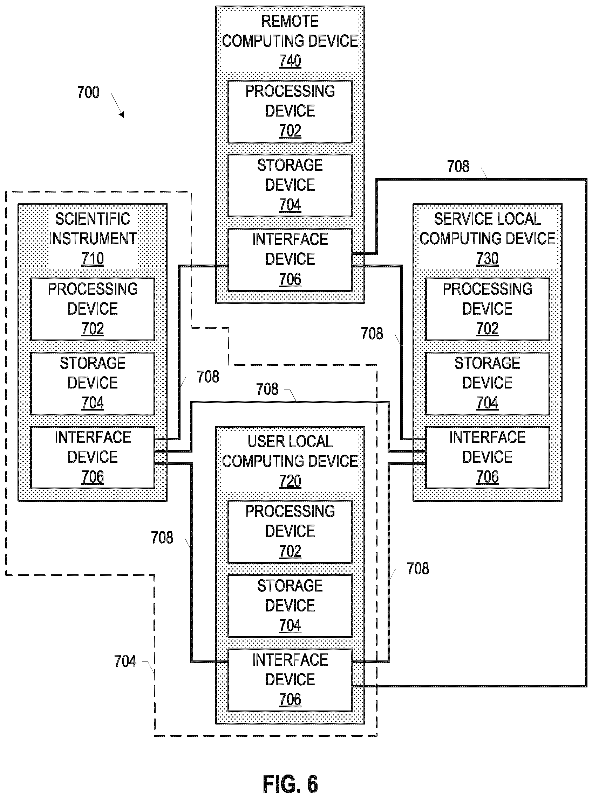

FIG. 2 is a block diagram of a scientific instrument support module 200 for performing support operations, in accordance with various embodiments. The scientific instrument support module 200 may be implemented, for example, via the server 128. The scientific instrument support module 200 may be implemented by circuitry (e.g., including electrical and/or optical components), such as a programmed computing device. The logic of the scientific instrument support module 200 may be included in a single computing device or may be distributed across multiple computing devices that are in communication with each other as appropriate. Examples of computing devices that may, singly or in combination, implement the scientific instrument support module 200 are discussed herein with reference to the computing device 600 of FIG. 5, and examples of systems of interconnected computing devices, in which the scientific instrument support module 200 may be implemented across one or more of the computing devices, is discussed herein with reference to the scientific instrument support system 700 of FIG. 6.

As illustrated in FIG. 2, the scientific instrument support module 200 may include instrument utilization logic 204, method creator logic 206, and data review logic 208. As used herein, the term "logic" may include an apparatus that is to perform a set of operations associated with the logic. For example, any of the logic elements included in the support module 200 may be implemented by one or more computing devices programmed with instructions to cause one or more electronic processing devices of the computing devices to perform the associated set of operations. In a particular embodiment, a logic element may include one or more non-transitory computer-readable media having instructions thereon that, when executed by one or more electronic processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more electronic processing devices. A module may not include all of the logic elements depicted in the associated drawing; for example, a module may include a subset of the logic elements depicted in the associated drawing when that module is to perform a subset of the operations discussed herein with reference to that module.

The instrument utilization logic 204 may be configured to acquire, for each of the plurality of scientific instrument groups 118, asset information associated with each IPC 104, such as, for example, the device(s) 108, the instrument 112, or other components associated with each IPC 104. The asset information may include an identifier of an instrument including a type identifier, a unique identifier, or any combination thereof. In various implementations, the asset information also includes a real-time data acquisition status, which may include a status of an operation currently being performed, previously performed, or pending performance by the device(s) 108, the IPC 104, the instrument 112, or a combination thereof. The asset information may also include device details associated with each device(s) 108 such as, for example, a device name, a device serial number, a device model number, a device firmware version, a device warranty expiration date, a device warranty status, a device service provider, a service status, a contract expiration date, or any combinations thereof. The asset information may also include other status logs associated with the device(s) 108, the IPC 104, and/or the instrument 112, such as, for example, error logs or operation history.

The instrument utilization logic 204 may also be configured to acquire, for each of the plurality of scientific instrument groups 118, reservation information. The reservation information includes scheduling data associated with the planned availability of each scientific instrument group 118. The reservation information may include, for example, a reservation start date and time, a reservation end date and time, a reservation assignee (e.g., a scientist or technician in the lab that owns the reservation), or the like. The reservation information may also include a project identifier associated with a reservation. The asset and reservation information acquired via the instrument utilization logic 204 may be stored in the database 124.

The method creator logic 206 may be configured to receive data associated with a sample of interest (e.g., information defining a type of the sample of interest (e.g., soil, a specific plant, a biological sample, or the like)), a desired analysis (e.g., one or more compounds of interest), or a combination thereof and generate a list of one or more recommended methods for performing scientific experiments with respect to the sample. The desired analysis may specify one or more chemical constituents and/or one or more biological constituents (i.e., compounds of interest) to identify in the sample (and, optionally quantities thereof), a characteristic of the sample (e.g., a pH of the sample, a concentration, a mass percent, a molar percent, a volume percent or ratio), a density, a hardness, a boiling point, a melting point, a volatility, a flammability, a toxicity, a reactivity, a half-life, or a combination thereof. In some embodiments, the desired analysis is optional, and the systems and methods described herein may be configured to output recommended methods as part of an open or untargeted screening or analysis of the sample. Similarly, in some embodiments, the sample type is optional, and the systems and methods described herein may be configured to output recommended methods for one or more specified compounds of interest without requiring a sample type input.

Each method included in the generated list may include one or more experiment parameters. The experiment parameters may specify a parameter associated with the sample, an instrument used to analyze the sample, processing of the results of the analysis, or the like. For example, the experiment parameters may include a preparation parameter, a liquid dispersion parameter, a solid dispersion parameter, one or more solvent parameters, one or more concentration parameters, one or more ratios, volume percent, mass percent, molar percent, temperatures, pressures, times, column types or parameters, or combinations thereof.

In some embodiments, the method creator logic 206 includes and applies a machine-learned model (e.g., one or more neural networks) trained to, based on an inputted type of sample, a desired analysis, or a combination thereof, generate a list of one or more recommended methods. Such a machine-learned model may be trained using data stored on the database 124. For example, a machine-learned model may be trained with information on previous methods applied by an instrument on the platform, methods provided by third parties (e.g., accredited methods, vendor-specific methods, third-party methods pulled from external sources, such as journals and whitepapers), and the like. In particular, as illustrated in FIG. 3, the logic 206 may use the acquisition method data 301, the processing method data 302, the accredited method data 303, the vendor-specific method data 304, the legacy method data 305, the company-focused method data 306, the third-party method data 307, the library data 308, or any combination thereof to recommend one or more methods and, in some embodiments, may use all or a subset of this data to train a model to generate such recommendations. The information used to train the model may associate a particular method (including various experiment parameters) with a particular type of sample and particular type of analysis (i.e., a linked data set). Accordingly, using such data as training data, allows the machine-learned model, once trained, to receive as input a type of sample, a desired analysis, or a combination thereof and output one or more recommended methods. For example, the model may be configured to output a list of methods (that may be ranked based on applicability to the inputs via, for example, a confidence score) and may use further interaction with the user to narrow down the list and create a method for the sample of interest. This interaction can serve as further feedback to the machine-learned model, which allows the model to provide improved recommendations during subsequent use.

The data stored in the database 124 and used to train the model may take various forms, including, for example, text data, binary data, raw files, analytical results data, or the like and may be compiled from various sources and linked for training purposes in various ways. For example, the data used to train the model may include a plurality of data sets, wherein each data set links a sample or sample type, one or more compounds of interest, and an experiment method. Such data may be compiled by the system from various sources. For example, historical methods implemented on the platform may be stored (e.g., as the acquisition method data 301, the processing method data 302, the accredited method data 303, the legacy method data 305, the company-focused method data 306, or a combination thereof) and may represent linked data set, wherein stored data regarding the historical execution of a method identifies the experiment parameters of the method and may be stored or linked to sample data managed via the platform and data results also managed by the platform. Accordingly, the connected platform creates a data repository where linked data sets are stored or can be established from the stored data collected and managed by the platform.

Alternatively or in addition, one or more sets of linked data sets may be created from other sources and with other types of data. For example, the library data 308 may include spectral libraries for various compounds of interests and associated experiment parameters and this data may be combined with historical data representing methods applied to samples to create a set of linked data for training purposes. Similarly, the library data 308 may include a database of compounds of interest and various identifiers or names for such compounds, and this data may be used by the system to translate or link historical data, other library data, third-party data, or the like to particular compounds of interest. The library data 308 may also include a database of instrument geometries, which may be used to establish experiment parameters for a particular method (e.g., instrument settings). As another example, the third-party method data 307 may include journals or other text-based descriptions of experiments, which the system may be configured to parse and establish a linked data set (e.g., from the text-based description and/or in combination with other data from other sources). Accordingly, the data used to train the model as described herein may be obtained from one or more sources (e.g., various databases) and may take various forms and may be linked to data from other sources to create the training data.

Alternatively or in addition, the method creator logic 206 may be configured to access stored method data and retrieve applicable method data based on one or more static rules applied by the method creator logic 206 (e.g., matching user inputs to historical method data accessible by the method creator logic 206). This configuration may similarly provide a user with a list of recommended methods but, in some embodiments, the list of recommended methods may be limited to available stored methods and, thus, unlike machine-learned model, may not be able to recommend a method for an input that is generated to match the inputted data. In some embodiments, the method creator logic 206 may use a combination of rules and machine learning to provide a recommended method. For example, in some embodiments, the method creator logic 206 may be configured to initially generate a list of recommended methods from historical data via one or more rules and may use machine-learned functionality (e.g., chat-based interaction) to further refine this list based on user interactions with the list of recommended methods. Other combinations are also possible.

Regardless of how stored data is used to generate one or more recommended methods, the method creator logic 206 may also take into account stored asset and reservation information. For example, as illustrated in FIG. 3, the method creator logic 206 may use data managed by the instrument utilization logic 204 (which may be provided from the instrument utilization logic 204 or accessed from data storage where the instrument utilization logic 204 stores data) to recommend methods that are applicable to the instruments available for use by the user for processing the sample (e.g. the type of instruments included in the laboratory, a status or availability of such instruments, the health or maintenance status of an instrument (e.g., a level of consumables available, or the like). Similarly, the method creator logic 206 may use instrument information managed by the instrument utilization logic 204 to recommend one or more methods optimized based on the available instruments. For example, the method creator logic 206 may use instrument information to recommend a method associated with the shortest timeframe to complete the scientific experiment, a method allowing an experiment to be performed soonest based on availability of one or more instruments 112, a method using the fewest number of instruments or devices to complete the scientific experiment, a least cost-intensive method of operation to perform the scientific experiment, or combinations thereof. Accordingly, the method creator logic 206 uses the availability of data associated with a connected platform devices to not only recommend a method for processing a particular sample of interest but also take into account what instruments and devices are actually available for processing the sample, which reduces further processing time and inefficient use of computing and instrument resources.

Such optimizations may be implemented via one or more stored rules applied via the method creator logic 206. Also, in some embodiments, as part of developing a method, a user can specify one or more optimization rules to be applied. For example, as part of these optimization rules, a user may specify whether they only want to receive recommended methods that could be implemented with available instruments, recommended methods regardless of whether the method could be implemented with available instruments, or a combination thereof (e.g., by turning on and off or other toggling one or more optimization rules). Providing recommended methods for use with available instruments improves efficiency as a user does not waste time considering a method that may not be possible given current laboratory resources. However, providing recommended methods that are not limited to available laboratory resources may allow a user (e.g., a laboratory manager) to assess types of laboratory resources that may be needed to accommodate current demands or understand how available laboratory resources may be limiting a laboratory's efficiency, accuracy, or the like. This data may be useful for making purchasing recommendations and overall laboratory management.

Also, in some embodiments, in addition to outputting a developed method (e.g., a list of one or more recommended methods with associated experiment parameters), the method creator logic 206 is also configured to automatically generate and issue one or more reservations for reserving one or more instruments, devices, or the like associated with a particular developed method. Accordingly, the method creator logic 206 may be configured to automatically reserve resources for a developed method. The method creator logic 206 may reserve such resources in response to receiving a selection from a user of a particular developed method. Alternatively, the method creator logic 206 may reserve resources for one or more developed methods in response to limiting the number of available methods. In some embodiments, the method creator logic 206 may transmit reservations to the instrument utilization logic 204 or separate logic configured to manage a schedule of an instrument, device, or the like.

The data review logic 208 may be configured to aid the system and/or a user in analyzing the results of a method (e.g., developed via the method creator logic 206). For example, the method creator logic 206 and/or the data review logic 208 may be configured to generate expected results associated with a method, and the method creator logic 206 and/or the data review logic 208 may be configured to compare the actual results of the method with the expected results, such as, for example, to identify issues or errors with the method, the sample, or the like. The expected results may include historical result data (e.g., stored in the database 124) in a raw form or a processed form (e.g., an average) associated with similar methods as the method used to process the sample. Alternatively or in addition, the expected results may include predicted (synthetic) results for a method, which may be generated using a machine-learned model trained with pairs of methods and associated data results (again, which may be stored in the database 124 as described above). In various implementations, data output from the data review logic 208 may be provided in a user interface and such data may include the expected results, the actual results of the method, a comparison of the same, or a combination thereof.

In some implementations, output from the data review logic 208 may be fed back to the method creator logic 206. For example, output from the data review logic 208 may be used as additional feedback or training data for a machine learned model implemented via the method creator logic 206. Alternatively or in addition, the method creator logic 206 may use the feedback to create a modified method. For example, based on the output from the data review logic 208, the method creator logic 206 may develop a new method that may generate results that better match expected results. The method creator logic 206 may develop a new method based on data from the data review logic 208, such as, for example, by developing a new method that uses alternative instruments 112 to reduce operating cost, reduce the timeframe to complete the one or more scientific experiments, prioritize the order of which one or more scientific experiments will be performed before various other scientific experiments within any given working day, or a combination thereof. Data associated with a rerun of a method may be feed back to the model as training data to further improve the recommendations output via the model.

Similarly, the method creator logic 206 may develop a new method based on data from the data review logic 208 that modifies one or more experiment parameters of the original method to better calibrate the developed method for expected results. As noted above, in some embodiments, the method creator logic 206 may be configured as a method converter that may translate a method associated with one vendor (e.g., one instrument vendor) for a different vendor, such as, for example, by comparing actual results to expected results and iteratively modifying the initially developed method until the actual results match the expected results.

FIG. 3 is a block diagram showing functionality of the method creator logic 206 in accordance with some embodiments. As illustrated in FIG. 3 and generally described above, the method creator logic 206 is communicatively connected to one or more data storage devices storing, for example, the acquisition method data 301, the processing method data 302, the accredited method data 303, the vendor-specific method data 304, the legacy method data 305, the company-focused method data 306, the third-party method data 307, the library data 308, the data review logic 208, or a combination thereof. As described above, the method creator logic 206 may use the accessible data to recommend one or more methods for processing a particular type of sample, which may be input by a user in one or more user interfaces. For example, the method creator logic 206 may use the data to identify a stored method applicable to a particular sample and/or may use the data to train one or more machine-learned models to output one or more recommended methods in response to inputted data (e.g., a type of sample, a desired analysis, or a combination thereof). As also noted above, the method creator logic 206 may also communicate with the instrument utilization logic 204 (and/or data managed by the logic 204) to recommend one or more methods based on instrument availability and/or optimization. Also, in some embodiments, the method creator logic 206 may issue reservations for instrument(s) via communication with the instrument utilization logic 204. Also, as illustrated in FIG. 3, the method creator logic 206 may communicate with the data review logic 208 to compare actual method results with expected results and, optionally, use the comparison to modify the originally-developed method (e.g., recommend a different method, recommend an adjustment to the originally-developed method), or a combination thereof. It should be understood that the functionality described herein as being performed by the logics 204, 206, and 208 may be distributed in various configurations.

The scientific instrument support methods disclosed herein may include interactions with a human user (e.g., via the user local computing device 720 discussed herein with reference to FIG. 6). These interactions may include providing information to the user (e.g., information regarding the operation of a scientific instrument such as the scientific instrument 710 of FIG. 6, information regarding a sample being analyzed or other test or measurement performed by a scientific instrument, information retrieved from a local or remote database, or other information) or providing an option for a user to input commands or other information. In some embodiments, these interactions may be performed through a graphical user interface (GUI) that includes a visual display on a display device (e.g., the display device 610 discussed herein with reference to FIG. 5) that provides outputs to the user and/or prompts the user to provide inputs (e.g., via one or more input devices, such as a keyboard, mouse, trackpad, or touchscreen, included in the other I/O devices 612 discussed herein with reference to FIG. 5). The scientific instrument support systems disclosed herein may include any suitable GUIs for interaction with a user.

FIG. 4 depicts an example GUI 500 that may be used in the performance of some, or all of the support methods disclosed herein, in accordance with various embodiments. As noted above, the GUI 500 may be provided on a display device (e.g., the display device 610 discussed herein with reference to FIG. 5) of a computing device (e.g., the computing device 600 discussed herein with reference to FIG. 5) of a scientific instrument support system (e.g., the scientific instrument support system 700 discussed herein with reference to FIG. 6), and a user may interact with the GUI 500 using any suitable input device (e.g., any of the input devices included in the other I/O devices 612 discussed herein with reference to FIG. 5) and input technique (e.g., movement of a cursor, motion capture, facial recognition, gesture detection, voice recognition, actuation of buttons, etc.).

The GUI 500 may include a data display region 502, a data analysis region 504, a scientific instrument management region 506, and a settings region 508. The particular number and arrangement of regions depicted in FIG. 4 is simply illustrative, and any number and arrangement of regions, including any desired features, may be included in a GUI 500.

The data display region 502 may display data generated by a scientific instrument (e.g., the scientific instrument 710 discussed herein with reference to FIG. 6).

The data analysis region 504 may display the results of data analysis (e.g., the results of analyzing the data illustrated in the data display region 502 and/or other data). In some embodiments, the data display region 502 and the data analysis region 504 may be combined in the GUI 500 (e.g., to include data output from a scientific instrument, and some analysis of the data, in a common graph or region).

The scientific instrument management region 506 may include options that allow the user to manage a scientific instrument (e.g., the scientific instrument 710 discussed herein with reference to FIG. 6).

The settings region 508 may include options that allow the user to manage the features and functions of the GUI 500 (and/or other GUIs) and/or perform common computing operations with respect to the data display region 502 and data analysis region 504 (e.g., saving data on a storage device, such as the storage device 604 discussed herein with reference to FIG. 5, sending data to another user, labeling data, etc.). For example, the settings region 508 may include a sorting option, a grouping option, a filtering option, and/or a search bar of a scheduler user interface.

As noted above, the scientific instrument support module 200 may be implemented by one or more computing devices. FIG. 5 is a block diagram of a computing device 600 that may perform some or all of the scientific instrument support methods disclosed herein, in accordance with various embodiments. In some embodiments, the scientific instrument support module 200 may be implemented by a single computing device 600 or by multiple computing devices 600. Further, as discussed below, a computing device 600 (or multiple computing devices 600) that implements the scientific instrument support module 200 may be part of one or more of the scientific instrument 710, the user local computing device 720, the service local computing device 730, or the remote computing device 740 of FIG. 6.

The computing device 600 of FIG. 5 is illustrated as having a number of components, but any one or more of these components may be omitted or duplicated, as suitable for the application and setting. In some embodiments, some or all of the components included in the computing device 600 may be attached to one or more motherboards and enclosed in a housing (e.g., including plastic, metal, and/or other materials). In some embodiments, some these components may be fabricated onto a single system-on-a-chip (SoC) (e.g., an SoC may include one or more electronic processing devices 602 and one or more storage devices 604). Additionally, in various embodiments, the computing device 600 may not include one or more of the components illustrated in FIG. 5, but may include interface circuitry (not shown) for coupling to the one or more components using any suitable interface (e.g., a Universal Serial Bus (USB) interface, a High-Definition Multimedia interface (HDMI) interface, a Controller Area Network (CAN) interface, a Serial Peripheral interface (SPI) interface, an Ethernet interface, a wireless interface, or any other appropriate interface). For example, the computing device 600 may not include a display device 610, but may include display device interface circuitry (e.g., a connector and driver circuitry) to which a display device 610 may be coupled.

The computing device 600 may include an electronic processing device 602 (e.g., one or more processing devices). As used herein, the term "electronic processing device" may refer to any device or portion of a device that processes electronic data from registers and/or memory to transform that electronic data into other electronic data that may be stored in registers and/or memory. The electronic processing device 602 may include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable electronic processing devices.

The computing device 600 may include a storage device 604 (e.g., one or more storage devices). The storage device 604 may include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 604 may include memory that shares a die with an electronic processing device 602. In such an embodiment, the memory may be used as cache memory and may include embedded dynamic random access memory (eDRAM) or spin transfer torque magnetic random access memory (STT-MRAM), for example. In some embodiments, the storage device 604 may include non-transitory computer readable media having instructions thereon that, when executed by one or more electronic processing devices (e.g., the electronic processing device 602), cause the computing device 600 to perform any appropriate ones of or portions of the methods disclosed herein.

The computing device 600 may include an interface device 606 (e.g., one or more interface devices 606). The interface device 606 may include one or more communication chips, connectors, and/or other hardware and software to govern communications between the computing device 600 and other computing devices. For example, the interface device 606 may include circuitry for managing wireless communications for the transfer of data to and from the computing device 600. The term "wireless" and its derivatives may be used to describe circuits, devices, systems, methods, techniques, communications channels, etc., that may communicate data through the use of modulated electromagnetic radiation through a nonsolid medium. The term does not imply that the associated devices do not contain any wires, although in some embodiments they might not. Circuitry included in the interface device 606 for managing wireless communications may implement any of a number of wireless standards or protocols, including but not limited to Institute for Electrical and Electronic Engineers (IEEE) standards including Wi-Fi (IEEE 82.11 family), IEEE 82.16 standards (e.g., IEEE 82.16-2005 Amendment), Long-Term Evolution (LTE) project along with any amendments, updates, and/or revisions (e.g., advanced LTE project, ultra mobile broadband (UMB) project (also referred to as "3GPP2"), etc.). In some embodiments, circuitry included in the interface device 606 for managing wireless communications may operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 606 for managing wireless communications may operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 606 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 606 may include one or more antennas (e.g., one or more antenna arrays) to receipt and/or transmission of wireless communications.

In some embodiments, the interface device 606 may include circuitry for managing wired communications, such as electrical, optical, or any other suitable communication protocols. For example, the interface device 606 may include circuitry to support communications in accordance with Ethernet technologies. In some embodiments, the interface device 606 may support both wireless and wired communication, and/or may support multiple wired communication protocols and/or multiple wireless communication protocols. For example, a first set of circuitry of the interface device 606 may be dedicated to shorter-range wireless communications such as Wi-Fi or Bluetooth, and a second set of circuitry of the interface device 606 may be dedicated to longer-range wireless communications such as global positioning system (GPS), EDGE, GPRS, CDMA, WiMAX, LTE, EV-DO, or others. In some embodiments, a first set of circuitry of the interface device 606 may be dedicated to wireless communications, and a second set of circuitry of the interface device 606 may be dedicated to wired communications.

The computing device 600 may include battery/power circuitry 608. The battery/power circuitry 608 may include one or more energy storage devices (e.g., batteries or capacitors) and/or circuitry for coupling components of the computing device 600 to an energy source separate from the computing device 600 (e.g., AC line power).

The computing device 600 may include a display device 610 (e.g., multiple display devices). The display device 610 may include any visual indicators, such as a heads-up display, a computer monitor, a projector, a touchscreen display, a liquid crystal display (LCD), a light-emitting diode display, or a flat panel display.

The computing device 600 may include other input/output (I/O) devices 612. The other I/O devices 612 may include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 600, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 600 may have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

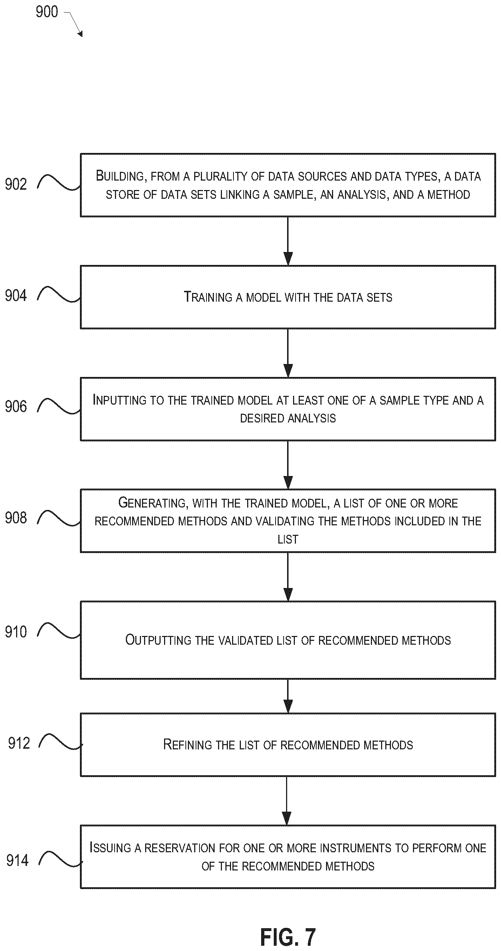

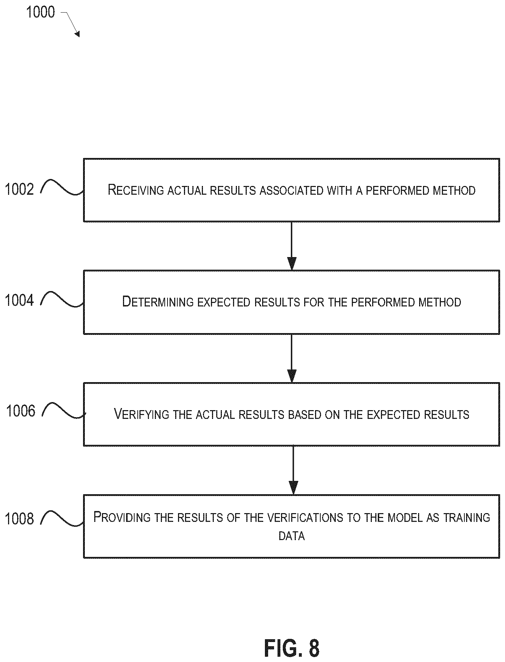

One or more computing devices implementing any of the scientific instrument support modules or methods disclosed herein may be part of a scientific instrument support system. FIG. 6 is a block diagram of an example scientific instrument support system 700 in which some or all of the scientific instrument support methods disclosed herein may be performed, in accordance with various embodiments. The scientific instrument support modules and methods disclosed herein (e.g., the scientific instrument support module 200 of FIG. 2 and the processes 900 and 1000 of FIGS. 7 and 8) may be implemented by one or more of the scientific instrument 710, the user local computing device 720, the service local computing device 730, or the remote computing device 740 of the scientific instrument support system 700.

Any of the scientific instrument 710, the user local computing device 720, the service local computing device 730, or the remote computing device 740 may include any of the embodiments of the computing device 600 discussed herein with reference to FIG. 5, and any of the scientific instrument 710, the user local computing device 720, the service local computing device 730, or the remote computing device 740 may take the form of any appropriate ones of the embodiments of the computing device 600 discussed herein with reference to FIG. 5.

The scientific instrument 710, the user local computing device 720, the service local computing device 730, or the remote computing device 740 may each include an electronic processing device 702, a storage device 704, and an interface device 706. The electronic processing device 702 may take any suitable form, including the form of any of the electronic processing devices 602 discussed herein with reference to FIG. 5, and the electronic processing devices 702 included in different ones of the scientific instrument 710, the user local computing device 720, the service local computing device 730, or the remote computing device 740 may take the same form or different forms. The storage device 704 may take any suitable form, including the form of any of the storage devices 604 discussed herein with reference to FIG. 5, and the storage devices 604 included in different ones of the scientific instrument 710, the user local computing device 720, the service local computing device 730, or the remote computing device 740 may take the same form or different forms. The interface device 706 may take any suitable form, including the form of any of the interface devices 606 discussed herein with reference to FIG. 5, and the interface devices 706 included in different ones of the scientific instrument 710, the user local computing device 720, the service local computing device 730, or the remote computing device 740 may take the same form or different forms.

The scientific instrument 710, the user local computing device 720, the service local computing device 730, and the remote computing device 740 may be in communication with other elements of the scientific instrument support system 700 via communication pathways 708. The communication pathways 708 may communicatively couple the interface devices 706 of different ones of the elements of the scientific instrument support system 700, as shown, and may be wired or wireless communication pathways (e.g., in accordance with any of the communication techniques discussed herein with reference to the interface devices 606 of the computing device 600 of FIG. 5). The particular scientific instrument support system 700 depicted in FIG. 6 includes communication pathways between each pair of the scientific instrument 710, the user local computing device 720, the service local computing device 730, and the remote computing device 740, but this "fully connected" implementation is simply illustrative, and in various embodiments, various ones of the communication pathways 708 may be absent. For example, in some embodiments, a service local computing device 730 may not have a direct communication pathway 708 between its interface device 706 and the interface device 706 of the scientific instrument 710, but may instead communicate with the scientific instrument 710 via the communication pathway 708 between the service local computing device 730 and the user local computing device 720 and the communication pathway 708 between the user local computing device 720 and the scientific instrument 710.

The scientific instrument 710 may include any appropriate scientific instrument, such as a scientific instrument 112 described above with respect to FIG. 1.

The user local computing device 720 may be a computing device (e.g., in accordance with any of the embodiments of the computing device 600 discussed herein) that is local to a user of the scientific instrument 710. In some embodiments, the user local computing device 720 may also be local to the scientific instrument 710, but this need not be the case; for example, a user local computing device 720 that is in a user's home or office may be remote from, but in communication with, the scientific instrument 710 so that the user may use the user local computing device 720 to manage and/or access data from the scientific instrument 710. In some embodiments, the user local computing device 720 may be a laptop, smartphone, or tablet device. In some embodiments the user local computing device 720 may be a portable computing device.

The service local computing device 730 may be a computing device (e.g., in accordance with any of the embodiments of the computing device 600 discussed herein) that is local to an entity that services the scientific instrument 710. For example, the service local computing device 730 may be local to a manufacturer of the scientific instrument 710 or to a third-party service company. In some embodiments, the service local computing device 730 may communicate with the scientific instrument 710, the user local computing device 720, and/or the remote computing device 740 (e.g., via a direct communication pathway 708 or via multiple "indirect" communication pathways 708, as discussed above) to receive data regarding the operation of the scientific instrument 710, the user local computing device 720, and/or the remote computing device 740 (e.g., the results of self-tests of the scientific instrument 710, calibration coefficients used by the scientific instrument 710, the measurements of sensors associated with the scientific instrument 710, etc.). In some embodiments, the service local computing device 730 may communicate with the scientific instrument 710, the user local computing device 720, and/or the remote computing device 740 (e.g., via a direct communication pathway 708 or via multiple "indirect" communication pathways 708, as discussed above) to transmit data to the scientific instrument 710, the user local computing device 720, and/or the remote computing device 740 (e.g., to update programmed instructions, such as firmware, in the scientific instrument 710, to initiate the performance of test or calibration sequences in the scientific instrument 710, to update programmed instructions, such as software, in the user local computing device 720 or the remote computing device 740, etc.). A user of the scientific instrument 710 may utilize the scientific instrument 710 or the user local computing device 720 to communicate with the service local computing device 730 to report a problem with the scientific instrument 710 or the user local computing device 720, to request a visit from a technician to improve the operation of the scientific instrument 710, to order consumables or replacement parts associated with the scientific instrument 710, or for other purposes.

The remote computing device 740 may be a computing device (e.g., in accordance with any of the embodiments of the computing device 600 discussed herein) that is remote from the scientific instrument 710 and/or from the user local computing device 720. In some embodiments, the remote computing device 740 may be included in a datacenter or other large-scale server environment. In some embodiments, the remote computing device 740 may include network-attached storage (e.g., as part of the storage device 704). The remote computing device 740 may store data generated by the scientific instrument 710, perform analyses of the data generated by the scientific instrument 710 (e.g., in accordance with programmed instructions), facilitate communication between the user local computing device 720 and the scientific instrument 710, and/or facilitate communication between the service local computing device 730 and the scientific instrument 710. The remote computing device may perform some or all of the functions of the server 128 (e.g., supporting the server-based platform 300).

In some embodiments, one or more of the elements of the scientific instrument support system 700 illustrated in FIG. 6 may not be present. Further, in some embodiments, multiple ones of various ones of the elements of the scientific instrument support system 700 of FIG. 6 may be present. For example, a scientific instrument support system 700 may include multiple user local computing devices 720 (e.g., different user local computing devices 720 associated with different users or in different locations). In another example, a scientific instrument support system 700 may include multiple scientific instruments 710, all in communication with service local computing device 730 and/or a remote computing device 740; in such an embodiment, the service local computing device 730 may monitor these multiple scientific instruments 710, and the service local computing device 730 may cause updates or other information may be "broadcast" to multiple scientific instruments 710 at the same time. Different ones of the scientific instruments 710 in a scientific instrument support system 700 may be located close to one another (e.g., in the same room) or farther from one another (e.g., on different floors of a building, in different buildings, in different cities, etc.). In some embodiments, a scientific instrument 710 may be connected to an Internet-of-Things (loT) stack that allows for management of the scientific instrument 710 through a web-based application, a virtual or augmented reality application, a mobile application, and/or a desktop application. Any of these applications may be accessed by a user operating the user local computing device 720 in communication with the scientific instrument 710 by the intervening remote computing device 740. In some embodiments, a scientific instrument 710 may be sold by the manufacturer along with one or more associated user local computing devices 720 as part of a local scientific instrument computing unit 712.

In some embodiments, different ones of the scientific instruments 710 included in a scientific instrument support system 700 may be different types of scientific instruments 710; for example, one scientific instrument 710 may be a mass spectrometer, while another scientific instrument 710 may be a liquid chromatography instrument. In some such embodiments, the remote computing device 740 and/or the user local computing device 720 may combine data from different types of scientific instruments 710 included in a scientific instrument support system 700.

FIG. 7 is a flow diagram of a method 900 of performing support operations, in accordance with various embodiments. Although the operations of the method 900 may be illustrated with reference to particular embodiments disclosed herein (e.g., the scientific instrument support modules 200 discussed herein with reference to FIG. 2 (e.g., the method creator logic 206), the GUI 500 discussed herein with reference to FIG. 4, the computing devices 600 discussed herein with reference to FIG. 5, and/or the scientific instrument support system 700 discussed herein with reference to FIG. 6), the method 900 may be used in any suitable setting to perform any suitable support operations. Operations are illustrated once each and in a particular order in FIG. 7, but the operations may be reordered and/or repeated as desired and appropriate (e.g., different operations performed may be performed in parallel, as suitable).

At 902, the method 900 includes building a data store of data sets linking experiment methods to samples and analyses. As described above, in some embodiments, the data store may be built from information managed for a connected platform of instruments (see, e.g., FIG. 1) and at least some of the data may include historical methods and associated sample types and analysis descriptions. The data store may be stored over one or more databases or other data storage devices. As also described above, the data may be obtained from various sources and may be in various forms. Also, the system (e.g., the method creator logic 206) may be configured to create sets of linked data based on data received from one or more sources. As noted above, in some embodiments, each data set links a sample (sample type), one or more compounds of interest, and a method, and the system may be configured to create these data sets based on historical data representing methods implemented via the instruments 112 included in the platform, journals or other text-based reports or publications (e.g., from third-parties), libraries (e.g., spectral libraries, instrument geometry libraries, compound libraries or databases, or the like), databases of established methods (e.g., as established by a company, as established by a vendor, as established according to accreditation process or entity, or the like), or the like and, in many situations, may establish links between data obtained from different sources and potentially in different formats.

At 904, the method 900 includes training a model with data (e.g., the data sets) from the data store. The model may include various neural networks or other types of machine-learned models and training the model may include training the model to output one or more recommended methods based on an inputted sample type, a desired analysis, or a combination thereof.

At 906, the method 900 includes inputting a sample type, a desired analysis, or a combination thereof into the model as trained. The input may be received from a user interface. As noted above, in some embodiments, only a sample type is input or only a desired analysis (e.g., one or more compounds of interest), are input. However, in other embodiments, the input includes a combination of these pieces of information. Also, in some embodiments, additional inputs are provided, such as, for example, priority information (e.g., how quickly are results needed), instrument optimization settings (e.g., should recommended methods be limited to those associated with available instruments or should recommended methods not be limited or filtered based on instrument availability), and the like.

At 908, the method 900 includes generating, with the trained model based on the inputted sample type, desired analysis, or combination thereof, a list of one or more recommended methods for analyzing the sample type. As described above, the model is trained to output one or more recommended methods based on provided input(s). As part of 908, the method 900 may also include validating the generated list such as, for example, by checking a recommended method for viability. This check may be performed by comparing a recommended method from the list to a database of methods, which may include accredited methods, historical methods, or the like. If the system cannot match a recommended method to a method from the database (e.g., as an identical match or a close match where a predetermined number of parameters match or fall within various thresholds), method may be removed from the list. Similarly, validating a method may include validating one or more experiment parameters included in a recommended method (e.g., by applying various rules) and removing a recommended method from the list that includes one or more parameters that fail such validations. In some embodiments, the validation results (e.g., whether and what methods were removed) may be provided as feedback to the model (i.e., as additional training information) to improve subsequent recommendations.

As noted above, in some embodiments, the list of recommended methods may be optimized for available scientific instrument resources, which may be collected and managed via the instrument utilization logic 904. For example, in some embodiments, the method creator logic 206 may create an initial list of recommended methods and modifying the list by obtaining information regarding available scientific instruments (e.g., instruments associated with a particular laboratory or set of laboratories) and modifying the list of one or more recommended methods based on the obtained information. Modifying the list may include removing a method, modifying a method, modifying a rank or score within the list, or a combination thereof. For example, as noted above, modifying the list may include removing a method based on what type of instruments are available for use (e.g., a method using a particular type of instrument that is not included in a laboratory or that is included in the laboratory but is reserved for the next month may be removed from the list, a method that is associated with a particular speed may be removed if no available scientific instrument can provide such a speed, etc.). The list of recommended methods may be output via a user interface.

At 910, the method 900 includes outputting the list of one or more recommended (and validated) methods for analyzing the sample type (e.g., within a user interface). As described in further detail below, the outputted list may include various rankings or scores and may allow a user to select a method from the list for execution (e.g., with or without modification). Optionally, at 912, the method 900 includes refining the list of recommended methods. In some embodiments, this refining is performed via interactions with a user (e.g., within a user interface) and may be configured as a chat-based interaction, wherein one or more prompts or questions may be presented to the user with the list of recommended methods wherein responses to the prompts/questions may be processed by the logic 206 to refine the list. For example, if a desired analysis was not initially input to the model, the user interface may prompt the user to input a desired analysis or may ask the user whether a particular analysis desired (e.g., starting with the most common or least common analysis represented within the list). Similarly, the user interface may prompt the user to specify one or more compounds of interest (e.g., a particular pesticide or list of pesticides), a particular regulatory requirement to be applied, a desired timing, or the like. The responses to such prompt may be used by the logic 206 to further refine the list of recommended methods, wherein refinement may include removal of a method from the list, modification of a method included in the list, a change to a ranking or score of the method within the list, or a combination thereof. In some embodiments, the refinement may continue until only a single method remains or until a user selects one or more methods from the list for execution. The refinement process may prompt a user for inputs, which may be applied as a filter to the recommended methods or used to rank or narrow the list to an initial method to implement. For example, in some embodiments, recommended methods may be associated with a rank or confidence score, which may be generated by the model and/or set or modified by a user. For example, in some embodiments, a method may be output by the model with an associated confidence score, which may be used to rank the methods, and, in some embodiments, a user may be able to modify the scores. In some embodiments, information collected during the refinement process may be provided as feedback to the model to improve subsequent method creation.

Optionally, at 914, the method 900 includes issuing a reservation for one or more instruments to implement one of the recommended methods (e.g., a method selected by a user). The reservation information may not only indicate the type of instrument being reserved and when such a reservation is requested but may also include one or more of the experiment parameters associated with the selected method. In this respect, method creation is further optimized by automatically setting various parameters of the method at the instruments, which makes more efficient use of resources and prevent potential human errors.

FIG. 8 is a flow diagram of a method 1000 of performing support operations, in accordance with various embodiments. Although the operations of the method 1000 may be illustrated with reference to particular embodiments disclosed herein (e.g., the scientific instrument support modules 200 discussed herein with reference to FIG. 2 (e.g., the method creator logic 206, the data review logic 208, or a combination thereof), the GUI 500 discussed herein with reference to FIG. 4, the computing devices 600 discussed herein with reference to FIG. 5, and/or the scientific instrument support system 700 discussed herein with reference to FIG. 6), the method 900 may be used in any suitable setting to perform any suitable support operations. Operations are illustrated once each and in a particular order in FIG. 8, but the operations may be reordered and/or repeated as desired and appropriate (e.g., different operations performed may be performed in parallel, as suitable). In some embodiments, the method 1000 may be performed after execution of the method 900 (e.g., for a selected recommended method as developed per the method creator logic 206). However, in other embodiments, the method 1000 may be performed regardless of how a performed method was developed.

At 1002, the method 1000 includes receiving actual results associated with a performed method. As noted above, the performed method may be one of the recommended methods developed according to the method 900, such as, for example, a method selected by a user from the list and including any modifications to the selected method made by the user (e.g., through a user interface as part of selecting a method from an outputted list). The actual results may be raw data or processed data and may represent data collected via one or more sensors of the instrument(s) used to perform the method.

At 1004, the method 1000 includes determining expected results for the performed method. As noted above, the expected results may include historical (actual) results generated using a method similar to (matching) the performed method. In other embodiments, the expected results may be predicted (synthetic results) that may be generated via one or more models trained using pairs of data associating an experiment method with results. The expected results may be in the same format as the actual results (e.g., raw form, processed form) or may, after being received, be processed to be in the same format as the actual results.

At 1006, the method 1000 includes verifying the actual results based on the expected results. This verification may include comparing the actual results and the expected results and identifying any differences (e.g., differences that exceeds a (configurable) threshold). Various forms of data analytics may be performed on the actual results and the expected results to identify whether there are any issues or errors with the performed method that may necessitate perform the method again (e.g., with a new sample, with new parameters, as a new/different method), or a combination thereof.

At 1008, the method 1000 includes providing the results of the verification to the model as further training data. For example, as noted above, in some embodiments, the verification results can be used to as part of a method converter configured to convert a known method for a known vendor to a similar method for a new vendor and the verification results may be part of an iterative process to develop the method for the new vendor. Similarly, in some embodiments, the results of the verification can be output by applying the results to the performed method to create a modified method that may be automatically implemented (e.g., in response to verification results satisfying particular criteria). Automatically implementing the modified method may include issuing new reservations for performing the modified method. Such reruns of a method (e.g., performed automatically or manually by a user) may be used as feedback to the model to improve subsequent method recommendations. In some embodiments, the results of the verification (or portions thereof) can also be provided to a user via one or more user interfaces.

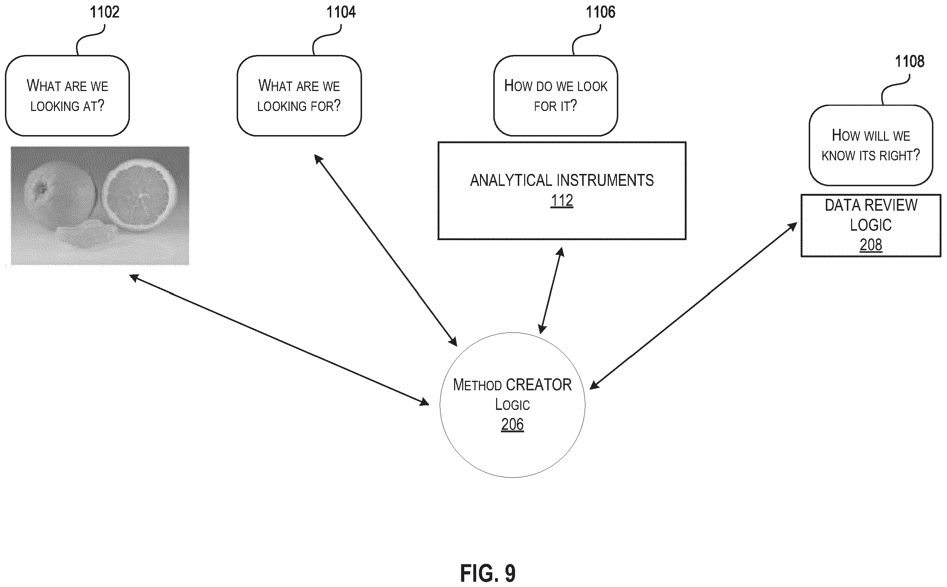

FIG. 9 is a block diagram schematically illustrating an example application of the methods 900 and 1000 using an example sample type (e.g., an orange). As illustrated in FIG. 9, in the example situation, the method creator logic 206 receives the inputted sample type and may output a list of recommended methods for the sample type (e.g., methods for analyzing orange samples). As noted above, the list of recommended methods may be optimized (e.g., refined) based on available instruments 112 and/or other optimization criteria (some of which may be specified as input) (see block 1106). For example, as illustrated in FIG. 9, a user may specify that the sample of interest is an orange sample and that the sample analysis has high priority (see block 1102). The logic 206 may use this input to limit and/or rank the list of recommended methods based on speed characteristics of the method (e.g., listing only those methods that can be completed in the next one to two days).

The method creator logic 206 may also refine the list of recommended methods based on a desired analysis (see block 1104) (which may be an initial input or a response to a prompt associated with providing an initial list). In the example of FIG. 9, the desired analysis may be identification of one or more pesticides according to a particular regulation (e.g., European).

As illustrated in FIG. 9, after a method is selected and performed, the actual results of the method can be processed (e.g., by the data review logic 208, such as through comparison with expected results) to confirm the results, and the results of this analysis can be fed back as training data for the model used for method creation (see block 1108). In addition, the analysis results can be used to analyze the results in an optimized manner to provide fast and intelligent data processing wherein relevant data is summarized or otherwise presented to a user for focused and efficient review. As noted above, in some embodiments, one or more machine learning techniques may be used to perform this review.

The results of the verification can be for various purposes (e.g., various analytics and/or associated user interfaces) and are not limited to verifying performance of a particular method. For example, the results may be used to track and control instrument utilization (e.g., identify instruments providing a high return on investment, schedule maintenance and planned downtime, identify what instruments are not running, etc.). Similarly, the results can be used to track reanalysis (e.g., re-performance of a method) and keep reanalysis to a low percentage of implemented methods. For example, keeping the analysis correct on the initial implementation of a method reduces costs and results in more efficient use of laboratory resources. Accordingly, tracking the analysis results can be used to provide a system overview or laboratory dashboard that can identify causes or reasons for reanalysis that can be corrected or addressed. Also, as illustrated in some of the example user interfaces provided herein, the verification results can be used to provide efficient and simplified user review in one or more user interfaces. For example, anomalies can be identified to allow analysis to spend time on data analysis and not bottlenecks associated with extracting or acquiring data. Similarly, by optimizing recommended methods based on available instruments, instruments can be used more efficiently, and various dashboards can be provided to track (e.g., in real-time) free instruments, how long it takes for a particular user to review match, and the like. All of this information not only results in improved management and usage of laboratory resources (e.g., improves ease of use of laboratory for analyzing a sample and increases confidence in results for even inexperienced lab users) but provides a high-level view or dashboard of a laboratory or portions thereof, which allow various labor mangers, schedulers, etc. to identify and correct issues.

The following paragraphs provide various examples of the embodiments disclosed herein. For example, according to one example disclosed above, e.g., in the summary section and/or in reference to any one or any combination of some or all of FIGS. 1-9, provided is a computer-implemented method for scientific instrument method creation. The method includes building a data store including a plurality of linked data sets, each of the linked data sets linking a predetermined experiment method defining a plurality of experiment parameters, a predetermined sample type, and a predetermined analysis; training a model using a machine-learning technique with the plurality of linked data set stored in the data store; inputting to the model, as trained, at least one selected from a group consisting of a sample type and a desired analysis; and outputting, from the model, a list of one or more recommended methods based on the at least one of the sample type and the desired analysis. The method may also include receiving actual experiment results collected via one or more scientific instructions, the actual experiment results associated with performance of a method from the list of one or more recommended methods; determining expected results for the performed methods; and verifying the actual experiment results based on expected results.

In some examples of the above method, building the data store includes obtaining data from at least one selected from a group consisting of a database of accredited methods, a database of historical methods, a database of vendor-specific methods, a spectral library, an instrument geometry library, a compound database, and database of research materials.

In some examples of any of the above methods, building the data store includes obtaining data in a plurality of formats, the plurality of formats including at least two selected from a group consisting of text data, binary data, and raw analytical data.

In some examples of any of the above methods, the desired analysis includes one or more compounds of interest.

In some examples of any of the above methods, outputting the list of one or more recommended methods for analyzing the sample type includes: obtaining information identifying a set of available scientific instruments, and modifying the list of one or more recommended methods based on the set of available scientific instruments. In some examples, obtaining the information identifying the set of available scientific instruments includes obtaining a type of each instrument in the set of available scientific instruments and wherein modifying the list of one or more recommended methods includes removing a method from the list of one or more recommended methods based on the type of each instrument in the set of available scientific instruments. In these examples or other examples, obtaining the information identifying the set of available scientific instruments includes obtaining a status of each instrument in the set of available scientific instruments and wherein modifying the list of one or more recommended methods includes removing a method from the list of one or more recommended methods based on the status of at least one instrument in the set of available scientific instruments. In these examples or other examples, obtaining the information identifying the set of available scientific instruments includes obtaining a speed of each instrument in the set of available scientific instruments and wherein modifying the list of one or more recommended methods includes removing a method from the list of one or more recommended methods based on the speed of at least one instrument in the set of available scientific instruments.

In some examples of any of the above methods, the method further includes issuing a reservation for one or more instruments for performing the method from the list of one or more recommended methods.

In some examples of any of the above methods, the method further includes validating a method included in the list of one or more recommended methods. In some example, validating the method included in the list of one or more recommended methods includes comparing the method with methods stored in a database of methods and removing the method from the list of one or more recommended methods in response to the method not matching one of the methods stored in the database. In these examples or other examples, validating the method included in the list of one or more recommended methods includes comparing at least one experiment parameter of the method with a threshold and removing the method from the list of one or more recommended methods in response to the experiment parameter not satisfying the threshold.

In some examples of any of the above methods, the method further includes refining the list of recommended methods. In some example, refining the list of recommended method includes providing a prompt a user for input and refining the list of recommended methods based on a response to the prompt.

In some examples of any of the above methods, the expected results include at least one selected from a group consisting of historical results and predicted synthetic results.

In some examples of any of the above methods, the method further includes providing the results of the verification to the model as training data.

In some examples of any of the above methods, the method further includes providing results of a rerun of the method to the model as training data.

In some examples of any of the above methods, the one or more experimental parameters include at least one selected from a group consisting of a matrix preparation, a solid dispersion preparation, a liquid dispersion preparation, a time, a temperature, a pressure, a concentration, and a column type.

In some examples of any of the above methods, the method further includes providing user feedback on the list of recommended methods to the model as training data. In some examples, the user feedback includes a score for a method included in the list of recommended methods.

According to another example disclosed above, e.g., in the summary section and/or in reference to any one or any combination of some or all of FIGS. 1-9, provided is a system for scientific instrument method creation. One example system includes non-transitory computer readable medium storing a data store including a plurality of linked data sets, each of the linked data sets linking a predetermined experiment method defining a plurality of experiment parameters, a predetermined sample type, and a predetermined analysis; and at least one electronic processing device. The at least one electronic processor is for (e.g., is configured to) training a model using a machine-learning technique with the plurality of linked data set stored in the data store; inputting to the model, as trained, at least one selected from a group consisting of a sample type and a desired analysis; and outputting, from the model, a list of one or more recommended methods based on the at least one of the sample type and the desired analysis. In some examples, the at least one electronic processing devices is also for receiving actual experiment results collected via one or more scientific instructions, the actual experiment results associated with performance of a method from the list of one or more recommended methods; determining expected results for the performed methods; and verifying the actual experiment results based on expected results.

In some examples of the above system, the desired analysis includes one or more compounds of interest.

In some examples of any of the above systems, to output, from the model, the list of one or more recommended methods, the electronic processing device is configured to: obtain information identifying a set of available scientific instruments and modify the list of one or more recommended based on the set of available scientific instruments. In these or other examples, to obtain the information identifying the set of available scientific instruments, the electronic processing device is configured to obtain a type of each instrument in the set of available scientific instruments. In these or other examples, to modify the list of one or more recommended methods, the electronic processing device is configured to remove a method from the list of one or more recommended methods based on the type of each instrument in the set of available scientific instruments. In these or other examples, to obtain the information identifying the set of available scientific instruments, the electronic processing device is configured to obtain a status of each instrument in the set of available scientific instruments. In these or other examples, to modify the list of one or more recommended methods, the electronic processing device is configured to remove a method from the list of one or more recommended methods based on the status of at least one instrument in the set of available scientific instruments. In these or other examples, to obtain the information identifying the set of available scientific instruments, the electronic processing device is configured to obtain a speed of each instrument in the set of available scientific instruments. In these or other examples, to modify the list of one or more recommended methods, the electronic processing device is configured to remove a method from the list of one or more recommended methods based on the speed of at least one instrument in the set of available scientific instruments.

In some examples of any of the above systems, the electronic processing device is further configured to issue a reservation for one or more instruments for performing the method from the list of one or more recommended methods.

In some examples of any of the above systems, the electronic processing device is further configured to validate a method included in the list of one or more recommended methods by comparing the method with methods stored in a database of methods and removing the method from the list of one or more recommended methods in response to the method not matching one of the methods stored in the database. In these or other examples, the electronic processing device is further configured to validate the method included in the list of one or more recommended methods by comparing at least one experiment parameter of the method with a threshold and removing the method from the list of one or more recommended methods in response to the experiment parameter not satisfying the threshold.

In some examples of any of the above systems, the electronic processing device is further configured to refine the list of recommended methods by providing a prompt to a user for input and refining the list of recommended methods based on a response to the prompt.

In some examples of any of the above systems, the expected results include at least one selected from a group consisting of historical results and predicted synthetic results.

In some examples of any of the above systems, the electronic processing device is further configured to provide the results of the verification to the model as training data.

In some examples of any of the above systems, the electronic processing device is further configured to provide results of a rerun of the method to the model as training data.

In some examples of any of the above systems, the plurality of experiment parameters include at least one selected from a group consisting of a matrix preparation, a solid dispersion preparation, a liquid dispersion preparation, a time, a temperature, a pressure, a concentration, and a column type.

In some examples of any of the above systems, the electronic processing device is configured to provide user feedback on the list of recommended methods to the model as training data. In these or other examples, the user feedback includes a score for a method included in the list of recommended methods.

According to another example disclosed above, e.g., in the summary section and/or in reference to any one or any combination of some or all of FIGS. 1-9, provided is non-transitory computer readable medium storing instructions that, when executed by one or more electronic processing devices, perform a set of functions. The set of functions includes building a data store including a plurality of linked data sets, each of the linked data sets linking a predetermined experiment method defining a plurality of experiment parameters, a predetermined sample type, and a predetermined analysis; training a model using a machine-learning technique with the plurality of linked data set stored in the data store; inputting to the model, as trained, at least one selected from a group consisting of a sample type and a desired analysis; and outputting, from the model, a list of one or more recommended methods based on the at least one of the sample type and the desired analysis. In some examples, the set of functions also includes receiving actual experiment results collected via one or more scientific instructions, the actual experiment results associated with performance of a method from the list of one or more recommended methods; determining expected results for the performed methods; and verifying the actual experiment results based on expected results.

In some examples of the above non-transitory computer readable medium, building the data store includes obtaining data from at least one selected from a group consisting of a database of accredited methods, a database of historical methods, a database of vendor-specific methods, a spectral library, an instrument geometry library, a compound database, and database of research materials.

In some examples of any of the above non-transitory computer readable medium, building the data store includes obtaining data in a plurality of formats, the plurality of formats including at least two selected from a group consisting of text data, binary data, and raw analytical data.

According to yet another example disclosed above, e.g., in the summary section and/or in reference to any one or any combination of some or all of FIGS. 1-9, provided is a computer-implemented method for scientific instrument method creation. The method includes building a data store including a plurality of linked data sets, each of the linked data sets linking a predetermined experiment method defining a plurality of experiment parameters, a predetermined sample type, and a predetermined analysis; and training a model using a machine-learning technique with the plurality of linked data set stored in the data store, the model, when trained, configured to receive at least one selected from a group consisting of a sample type and a desired analysis and output a list of one or more recommended methods based on the at least one of the sample type and the desired analysis.

According to yet a further example disclosed above, e.g., in the summary section and in reference to any one or any combination of some or all of FIGS. 1-9, provided is a computer-implemented method for scientific instrument method creation. The method includes inputting to a model, trained using linked data linking a predetermined experiment method defining a plurality of experiment parameters, a predetermined sample type, and a predetermined analysis, at least one selected from a group consisting of a sample type and a desired analysis; and outputting, from the model, a list of one or more recommended methods based on the sample type or the desired analysis.

In some examples of any of the above method, the method further includes receiving actual experiment results collected via one or more scientific instructions, the actual experiment results associated with performance of a method from the list of one or more recommended methods; determining expected results for the performed methods; and verifying the actual experiment results based on expected results.

## Claims

1. A system for scientific instrument method creation, the system comprising:
non-transitory computer readable medium storing a data store including a plurality of linked data sets, each of the linked data sets linking a predetermined experiment method defining a plurality of experiment parameters, a predetermined sample type, and a predetermined analysis; and
an electronic processing device configured to:
train a model using a machine-learning technique with the plurality of linked data set stored in the data store;
input to the model, as trained, at least one selected from a group consisting of a sample type and a desired analysis;
output, from the model, a list of one or more recommended methods based on the at least one of the sample type and the desired analysis;
receive actual experiment results collected via one or more scientific instructions, the actual experiment results associated with performance of a method from the list of one or more recommended methods;
determine expected results for the performed methods; and
verify the actual experiment results based on the expected results.

2. The system of claim 1, wherein the desired analysis includes one or more compounds of interest.

3. The system of claim 1, wherein, to output, from the model, the list of one or more recommended methods, the electronic processing device is configured to:
obtain information identifying a set of available scientific instruments, and
modify the list of one or more recommended methods based on the set of available scientific instruments.

4. The system of claim 3, wherein, to obtain the information identifying the set of available scientific instruments, the electronic processing device is configured to obtain a type of each instrument in the set of available scientific instruments, and wherein, to modify the list of one or more recommended methods, the electronic processing device is configured to remove a method from the list of one or more recommended methods based on the type of each instrument in the set of available scientific instruments.

5. The system of claim 3, wherein, to obtain the information identifying the set of available scientific instruments, the electronic processing device is configured to obtain a status of each instrument in the set of available scientific instruments, and wherein, to modify the list of one or more recommended methods, the electronic processing device is configured to remove a method from the list of one or more recommended methods based on the status of at least one instrument in the set of available scientific instruments.

6. The system of claim 3, wherein, to obtain the information identifying the set of available scientific instruments, the electronic processing device is configured to obtain a speed of each instrument in the set of available scientific instruments, and wherein, to modify the list of one or more recommended methods, the electronic processing device is configured to remove a method from the list of one or more recommended methods based on the speed of at least one instrument in the set of available scientific instruments.

7. The system of any one of claim 1, claim 2, or claim 3, wherein the electronic processing device is further configured to issue a reservation for one or more instruments for performing the method from the list of one or more recommended methods.

8. The system of any one of claim 1, claim 2, or claim 3, wherein the electronic processing device is further configured to validate a method included in the list of one or more recommended methods by comparing the method with methods stored in a database of methods and removing the method from the list of one or more recommended methods in response to the method not matching one of the methods stored in the database.

9. The system of any one of claim 1, claim 2, or claim 3, wherein the electronic processing device is further configured to validate the method included in the list of one or more recommended methods by comparing at least one experiment parameter of the method with a threshold and removing the method from the list of one or more recommended methods in response to the experiment parameter not satisfying the threshold.

10. The system of any one of claim 1, claim 2, or claim 3, wherein the electronic processing device is further configured to refine the list of recommended methods by providing a prompt to a user for input and refining the list of recommended methods based on a response to the prompt.

11. The system of any one of claim 1, claim 2, or claim 3, wherein the expected results include at least one selected from a group consisting of historical results and predicted synthetic results.

12. The system of any one of claim 1, claim 2, or claim 3, wherein the electronic processing device is further configured to provide the results of the verification to the model as training data.

13. The system of any one of claim 1, claim 2, or claim 3, wherein the electronic processing device is further configured to provide results of a rerun of the method to the model as training data.

14. The system of any one of claim 1, claim 2, or claim 3, wherein the plurality of experiment parameters include at least one selected from a group consisting of a matrix preparation, a solid dispersion preparation, a liquid dispersion preparation, a time, a temperature, a pressure, a concentration, and a column type.

15. The system of any one of claim 1, claim 2, or claim 3, wherein the electronic processing device is configured to provide user feedback on the list of recommended methods to the model as training data, and wherein the user feedback includes a score for a method included in the list of recommended methods.
